# FASCICULE DE BREVET EUROPEEN

(11) **EP 2 238 897 B1**
(45) Date de publication et mention de la délivrance du brevet: **13.04.2011**
(21) Numéro de dépôt: 10159093.3
(22) Date de dépôt: 06.04.2010
(51) Int. Cl.: A61B 5/0402, A61B 5/042, A61B 5/04, A61N 1/37, G06N 3/02, G06F 17/00

(54) **Dispositif médical actif comprenant des moyens de reconstruction d'un électrocardiogramme de surface à partir d'un électrogramme endocavitaire**
Aktive medizinische Vorrichtung, die Mittel zur Rekonstruktion eines Oberflächenelektrokardiogramms auf der Grundlage einer endokavitären Aktionsstromkurve umfasst
Active medical device including means for reconstructing a surface electrocardiogram using an intracardiac electrogram

(30) Priorité: 06.04.2009 FR 0901662
(43) Date de publication de la demande: 13.10.2010
(73) Titulaire: Sorin CRM SAS, 92541 Montrouge (FR)
(72) Inventeur: Carrault, Guy, 35510, Cesson-Sévigné (FR); Hernandez, Alfredo, 35700, RENNES (FR); Kachenoura, Amar, 35000, RENNES (FR); Poree, Fabienne, 35000, RENNES (FR)
(74) Mandataire: Dupuis-Latour, Dominique

(56) Documents cités:
- EP-A- 0 784 996
- US-A1- 2008 013 747
- US-B1- 6 572 560
- C: VASQUEZ, A. HERNANDEZ, F. MORA, G. CARRAULT, G. PASSARIELLO: "Atrial Activity Enhancement by Wiener Filtering Using an Artifical Neural Network" IEEE TRANSACTIONS ON BIOMEDICAL ENGINEERING, vol. 48, no. 8, août 2001 (2001-08), pages 940-944, XP002549347

## Description

L'invention a trait au domaine des "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

L'invention concerne plus particulièrement une technique de traitement des signaux représentatifs des potentiels cardiaques de dépolarisation du myocarde, signaux recueillis par des électrodes épicardiques ou endocavitaires de stimulation ou de détection ou de défibrillation des oreillettes ou des ventricules droits et gauches de ces dispositifs implantés.

Plus précisément, l'invention propose un dispositif comprenant des moyens permettant de reconstruire au moins un électrocardiogramme de surface (ECG), notamment sur différentes dérivations, à partir d'au moins un électrogramme endocavitaire ou épicardique (EGM).

Les signaux EGM sont des signaux recueillis par des électrodes placées sur les sondes endocavitaires ou épicardiques implantées avec le dispositif. Ces signaux, directement issus de l'activité électrique des cellules cardiaques, fournissent une information très riche pour évaluer l'état du patient. De fait, après amplification, numérisation et filtrage, ils sont principalement utilisés pour piloter le stimulateur cardiaque et pour diagnostiquer certains troubles du rythme nécessitant le déclenchement automatique d'une thérapie antitachycardique, antibradycardique ou de resynchronisation interventriculaire, par mise en oeuvre d'algorithmes élaborés d'analyse et de prise de décision.

En revanche, lorsqu'il s'agit d'analyser de façon subjective le rythme pour poser un diagnostic ou pour réajuster les paramètres d'un implant, les médecins préfèrent, en pratique, interpréter les informations d'électrocardiogramme de surface (ECG), qui permettent de visualiser de façon directe un certain nombre de facteurs importants (largeur du QRS, ... ) et d'apprécier l'évolution d'une insuffisance cardiaque.

En effet, les signaux ECG et EGM, bien qu'ils aient la même source (l'activité électrique du myocarde), se présentent visuellement de façon assez différente : l'EGM recueilli par la prothèse cardiaque fournit une information locale sur l'activité électrique d'un groupe de cellules cardiaques, tandis que l'ECG apparaît sous la forme d'une information plus globale, influencée par la propagation du signal électrique entre le myocarde et la surface du corps, et par un certain nombre de différences morphologiques et pathologiques. Ainsi, l'affichage des signaux EGM n'est pas parlant pour un médecin habitué à interpréter de longue date des ECG de surface.

Ce sont également les signaux ECG qui sont enregistrés sur une longue période en ambulatoire par les dispositifs Holter, pour être ensuite traités et analysés afin d'évaluer l'état clinique du patient et diagnostiquer le cas échéant un trouble du rythme cardiaque.

De ce fait, lorsqu'un patient porteur d'un implant vient voir son médecin pour une visite de contrôle, celui-ci utilise en pratique deux appareils, à savoir un enregistreur ECG et un programmateur d'implant. Pour recueillir l'ECG, le praticien colle un certain nombre d'électrodes sur le torse du patient, de manière à définir les douze dérivations usuelles correspondant à autant de signaux ECG différents. Le programmateur externe est, quant à lui, utilisé pour contrôler certains paramètres de fonctionnement de l'implant (par exemple la durée de vie de la pile), télécharger des données enregistrées dans la mémoire de l'implant, et éventuellement modifier le paramétrage de ce dernier, y télécharger une version modifiée du logiciel de commande, etc.

La consultation chez le médecin requiert donc généralement deux appareils différents, ainsi qu'une manipulation pour la pose des électrodes de surface et le recueil des signaux ECG.

Le recours à ces deux appareils implique de plus le déplacement du patient dans un centre spécialement équipé, avec pour conséquence le plus souvent un espacement des visites de contrôle et donc un moindre suivi du patient.

Pour remédier à ces inconvénients, on a cherché à développer des algorithmes de reconstruction de l'ECG de surface à partir des signaux EGM, c'est-à-dire à partir des signaux directement délivrés par la prothèse implantée.

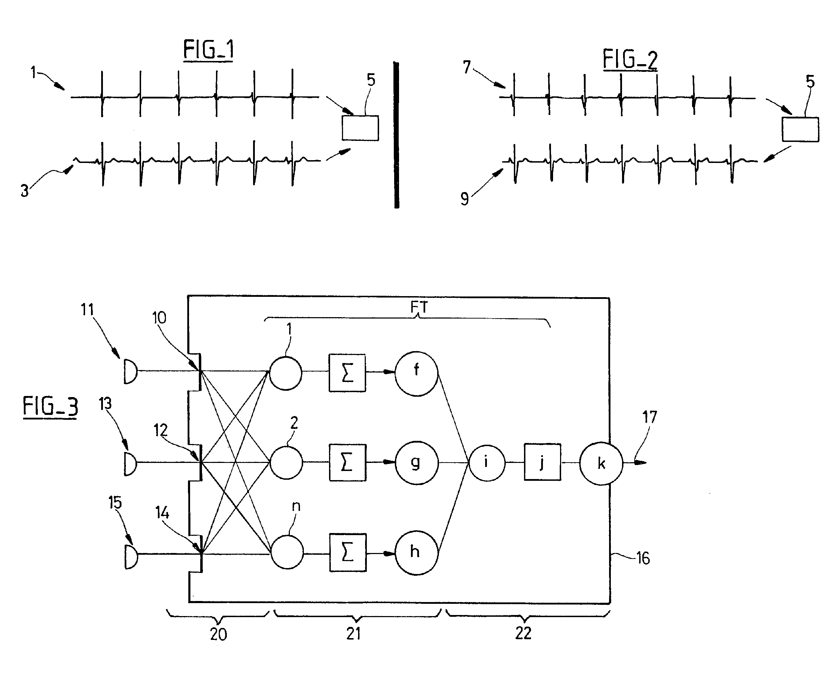

Divers algorithmes de reconstruction de l'ECG de surface à partir des signaux EGM ont été proposés en faisant appel à des réseaux de neurones, dont le fonctionnement est décrit ci-dessous en référence aux figures 1 et 2 illustrant, respectivement, l'étape de développement d'une fonction de transfert d'un réseau de neurones et l'étape de calcul, ou de reconstruction d'un signal ECG, au moyen de cette fonction de transfert.

Ainsi, sur la figure 1 sont représentés un signal EGM 1 et un signal ECG 3 transmis au réseau 5 de neurones afin que ce dernier apprenne, c'est-à-dire développe, une fonction de transfert 5 délivrant le signal ECG 3 en sortie lorsque le signal EGM 1 est fourni en entrée.

Lorsque cet apprentissage est effectué, on peut alors reconstituer un signal ECG 9 (figure 2) comme signal de sortie de ce réseau 5 de neurones après que sa fonction de transfert 5 ait traité un signal EGM 7.

A titre d'exemple, le EP 0 784 996 A1 (=US-A-5 740 811, Hedberg et al.) prévoit de synthétiser ainsi un signal ECG de surface par combinaison d'une pluralité de signaux EGM au moyen d'un réseau de neurones et/ou d'une logique floue et/ou d'un circuit sommateur, après apprentissage effectué par un algorithme du type *feedforward.*

Un tel réseau 16 est schématiquement représenté sur la figure 3. Typiquement, un tel réseau est constitué d'un ensemble d'entrées 20, d'une couche de neurones dite "cachée" ou couche "interne" 21, les entrées et les sorties de cette couche n'étant pas en liaison avec les données EGM et les données de sortie ECG, et d'une couche de neurones de sortie 22. La fonction de transfert est calculée dans la couche caché 21 et dans la couche de sortie 22. Ce réseau reçoit à ses différentes entrées 10, 12 et 14 des signaux EGM issus de différentes dérivations 11, 13 ou 15. Ces signaux EGM sont utilisés par un réseau 16 de neurones ayant préalablement développé une fonction de transfert FT, comprenant des sous-fonctions f, g, ... k, délivrant un signal 17 de sortie reproduisant un signal ECG reconstitué en fonction de signaux EGM acquis.

Pour ce faire, ce type de réseau 16 apprend, comme déjà décrit en référence aux figures 1 et 2, une fonction de transfert FT par l'exploitation d'une base d'apprentissage propre à un gabarit, c'est-à-dire à un type de classe de signal. De fait, l'apprentissage du réseau neuronal se fait entre un seul battement ECG et un seul battement EGM.

Or une telle solution n'est pas robuste. À titre d'exemple, des modifications dans l'intervalle du QT à l'effort (raccourcissement du QT) ne seront vraisemblablement pas prises en compte.

En outre, si un battement de morphologie nouvelle est acquis (extrasystole ventriculaire, battement jonctionnel, arythmie d'origine ventriculaire,...) il correspond alors à un type ou gabarit inconnu. Or un tel réseau de neurones ne peut pas transposer son apprentissage sur un gabarit connu, pour la synthèse d'un type ou gabarit inconnu.

De plus, la reconstruction se fait battement par battement et nécessite une étape intermédiaire pour concaténer les battements reconstruits, ce choix posant deux problèmes majeurs relatifs à la longueur de la fenêtre d'analyse et aux traitements des événements prématurés.

Il nécessite aussi une étape préliminaire de détection des QRS qui peut engendrer des défauts de détection et, de ce fait, de fausses reconstructions.

Finalement, elle nécessite au moins deux dérivations EGM, et donc a minima la présence de deux électrodes de recueil ainsi qu'une électrode de référence (boîtier de l'appareil), ou bien trois électrodes de recueil. L'invention a pour but de remédier à ces différents inconvénients en proposant un dispositif médical actif du type connu d'après le EP 0 784 996 A1 précité et correspondant au préambule de la revendication 1, comprenant :
- des moyens d'acquisition d'au moins un signal EGM échantillonné d'électrogramme endocavitaire à partir d'au moins une dérivation endocavitaire ou épicardique, à l'aide d'une pluralité d'électrodes endocavitaires,
- des moyens de calcul d'au moins un signal ECG d'électrocardiogramme de surface reconstitué, grâce au traitement des échantillons

EGM acquis, par une fonction de transfert d'un réseau de neurones.

De façon caractéristique de l'invention, le réseau de neurones est un réseau du type temps-retard destiné à traiter simultanément au moins un signal EGM, formé par une première séquence desdits échantillons acquis, et au moins une version retardée de ce signal EGM, formée par une seconde séquence desdits échantillons acquis distincte de la première séquence, de façon à fournir ledit signal ECG reconstitué à partir du signal EGM et de sa version retardée.

L'utilisation de tels réseaux temps-retard dans le domaine médical est connue et décrite par exemple par C. Vasquez et al. Atrial Activity Enhancement by Wiener Filtering Using an artificial Neural Network, IEEE Transactions on Biomedical Engineering, Vol. 48, No. 8, août 2001, pp. 940-944, ainsi que dans les US 2008/013747 A1 et US 6 572 560 B1. Il n'est toutefois jamais suggéré d'utiliser cette technique pour générer un signal ECG d'électrocardiogramme de surface reconstitué à partir de signaux EGM directement délivrés par une prothèse implantée.

Un tel dispositif présente de nombreux avantages grâce au traitement d'informations temporelles fournies par l'utilisation de différentes séquences d'échantillons issus d'un même signal EGM.

Notamment, un tel dispositif peut apprendre une nouvelle fonction de transfert de telle sorte qu'il délivre un ECG reconstitué acceptable en présence de battements nouveaux non appris (extrasystole ventriculaire, battement ou échappement jonctionnel, arythmie d'origine ventriculaire,...). Ainsi, le réseau peut appliquer la fonction de transfert qu'il a calculée lors de l'apprentissage pour fournir un résultat vraisemblable. En d'autres termes, un réseau temps-retard présente ainsi une capacité de généralisation plus forte qu'un réseau *feed-forward* selon l'art antérieur.

De plus, l'invention présente l'avantage de permettre, à partir d'une seule dérivation EGM, la reconstruction de signaux correspondant aux douze dérivations ECG.

Finalement, il convient de noter qu'un tel dispositif peut être mis en oeuvre avec des réseaux de neurones fonctionnant selon des modes SISO, MISO ou MIMO tels que définis ultérieurement.

Diverses formes de mises en oeuvre avantageuses sont énoncées dans les sous-revendications.

On va maintenant décrire un exemple de mise en oeuvre d'un dispositif conforme à l'invention, en référence aux dessins annexés où les mêmes références numériques désignent d'une figure à l'autre des éléments identiques ou fonctionnellement semblables.
Les figures 1 et 2, déjà décrites, illustrent l'étape d'apprentissage et l'étape de reconstruction respectivement mises en oeuvre pour un réseau de neurones,
La figure 3, déjà décrite, représente schématiquement un réseau de neurones mis en oeuvre pour la reconstruction d'un ECG selon une approche feed-forward conforme à l'art antérieur,
Les figures 4, 5 et 6 représentent schématiquement des mises en oeuvre de réseaux de neurones conformes à l'invention,
Les figures 7 et 8 sont des schémas fonctionnels de réseaux de neurones conformes à l'invention,
Les figures 9, 10, 11 et 12 sont respectivement des représentations d'un signal EGM, d'un signal ECG simultanément acquis et présentant un doublet ventriculaire, d'un signal ECG reconstruit selon l'art antérieur et d'un signal ECG reconstruit selon l'invention,
Les figures 13, 14 et 15 sont respectivement des représentations d'un signal EGM et d'un signal ECG simultanément acquis, et d'un signal ECG reconstruit selon l'invention vis-à-vis de battements d'origine sinusale,
Les figures 16, 17 et 18 sont respectivement des représentations d'un signal EGM et d'un signal ECG simultanément acquis, et d'un signal ECG reconstruit selon l'invention vis-à-vis de battements à morphologies multiples, et
Les figures 19, 20 et 21 sont respectivement des représentations d'un signal EGM et d'un signal ECG simultanément acquis, et d'un signal ECG reconstruit selon l'invention vis-à-vis de battements présentant un doublet ventriculaire.

On va maintenant décrire un exemple de réalisation du dispositif de l'invention, les termes "ECG" ou "EGM" étant utilisés de façon analogue aux termes "signal ECG" ou "signal EGM".

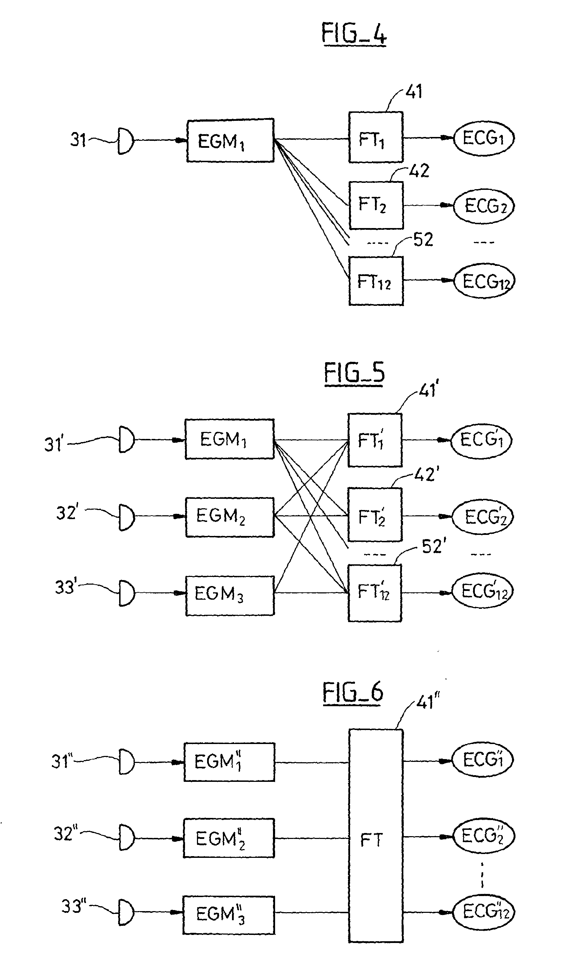

L'invention utilise une approche spécifique pour chaque patient à base d'un réseau de neurones du type temps-retard. Un tel réseau permet notamment de reconstruire un ECG à partir de signaux d'électrogramme endocavitaire (EGM) issus d'une dérivation endocavitaire ou épicardique, ces signaux étant acquis via une électrode endocavitaire ou épicardique. En référence aux figures 4, 5 et 6 sont schématiquement représentées trois variantes pour la mise en oeuvre de réseaux conformes à l'invention, à savoir :
- selon une première variante, une unique dérivation 31 (figure 4) délivre un signal EGM1 à des réseaux 41, 42, ...52 conformes à l'invention. Dans ce cas, chacun de ces réseaux 41, 42, ...52 utilise sa fonction de transfert FT1, FT2, ...ou FT12 pour fournir en sortie des signaux ECG1, ECG2, ..., ECG12 reconstitués correspondant, chacun, à une dérivation externe possible. Dans ce cas, chaque réseau 41, 42, ... 52 fonctionne selon une approche SISO, pour "Single Input Single Output".
- selon une seconde variante, une pluralité de signaux EGM1', EGM2' et EGM3' (figure 5), issus de dérivations 31', 32' ou 33' distinctes, est traitée par chaque réseau 41', 42', ...52', chacun de ces réseaux 41', 42', ... 52' utilisant sa fonction FT1', FT2', ... FT12' pour fournir en sortie des signaux ECG1', ECG2', ...ou ECG12' reconstitués correspondant, chacun, à une dérivation externe possible. Dans ce cas, chacun de ces réseaux 41', 42', ...52' fonctionne selon une approche MISO pour "Multiple Input Single Output".
- selon une troisième variante, une pluralité de signaux EGM1", EGM2" et EGM3" (figure 6), issus de dérivations 31', 32' ou 33' distinctes, est traitée par un réseau 41" utilisant sa fonction FT pour fournir en sortie des signaux ECG1", ECG2", ...et ECG12" reconstitués correspondant, chacun, à une dérivation externe possible. Dans ce cas, le réseau 41" fonctionne selon une approche MIMO pour "Multiple Input Multiple Output".

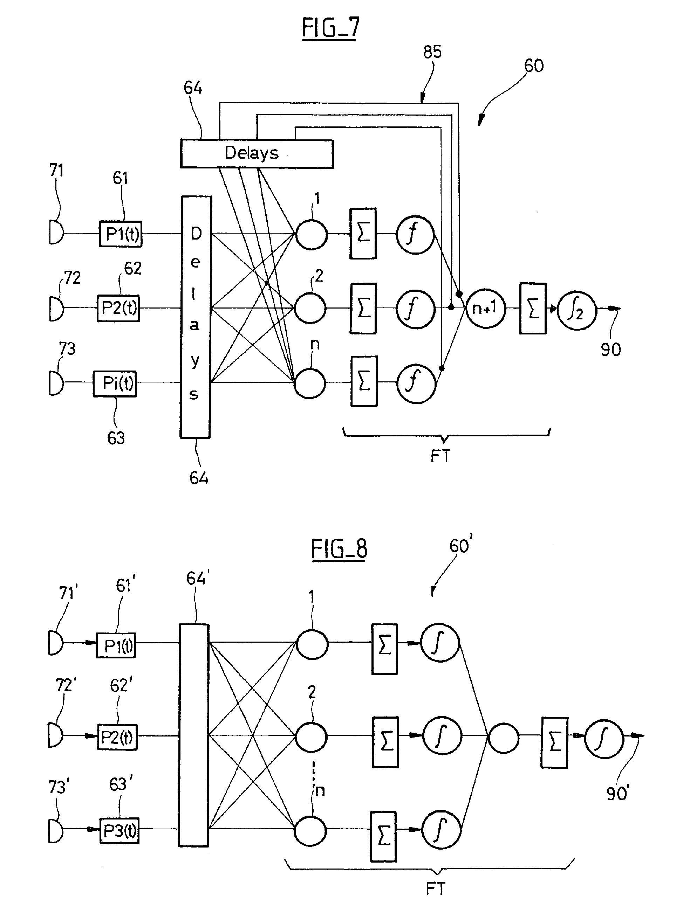

Conformément à l'invention, chacun de ces réseaux 41, 42, ..., 52, 41', 42', ..., 52' ou 41" est du type temps-retard, par exemple tel que décrit ci-dessous en référence aux figures 7 ou 8.

Selon une première variante de l'invention représentée à la figure 7, un réseau temps-retard 60 est du type récurrent. A cet effet, il comprend :
- des entrées 61, 62 et 63 recevant des signaux EGM acquis sur différentes dérivations 71, 72 et 73,
- des voies récurrentes 85, recevant des signaux traités par la couche cachée de ce réseau,
- des mémoires tampons 64 permettant d'introduire un temps de retard sur les signaux EGM traités et sur les voies récurrentes 85, et
- une fonction de transfert FT permettant de traiter les signaux EGM acquis, sur les dérivations 71, 72 et 73, et les versions retardées de ces signaux de façon à fournir un signal ECG 90 reconstitué.

Dans cette description de l'invention, chaque signal traité - et sa version retardée - est constitué par une séquence d'échantillons, c'est-à-dire de mesures, extraits parmi les échantillons formant un signal EGM acquis. Plus précisément, les réseaux de neurones conformes à l'invention traitent simultanément au moins un signal EGM formé par une première séquence des échantillons acquis et au moins une version retardée de ce signal EGM, formée par une seconde séquence desdits échantillons acquis distincte de la première séquence.

Dans une première réalisation, le signal et sa version retardée correspondent à deux séquences dont les échantillons sont décalés d'un pas p de l'ordre 0.01 s, ou plus généralement compris entre 0.001 s et 0.1 s.

Ainsi, en considérant un signal échantillonné comprenant deux cents échantillons (1, 2, 3, 4, 5, ...100, 101, 102, ...200), la première séquence peut comprendre les échantillons (1, 2, 3, 4, 5, 6, 7, 8, 9, ...100) tandis que la seconde séquence comprend les échantillons (d+1, d+2, ... d+100), avec d= p * fe, où fe est une fréquence d'échantillonnage et p est le pas de décalage entre séquences.

Par exemple dans le cas où p=0.01 s, si fe=100 Hz alors d=1. Selon un autre exemple, toujours dans le cas où p=0.01 s, si fe=1 kHz alors d=10. Selon un troisième exemple si p=0.02 s, et si fe=1 kHz alors d=20.

Il est clair que l'invention peut être mise en oeuvre en considérant, pour un signal, une pluralité de versions retardées présentant différents décalages entre les échantillons d'une même séquence et/ou entre les échantillons de différentes séquences.

En référence à la figure 8 est représentée une autre variante de l'invention mettant en oeuvre un réseau temps-retard 60' ne présentant pas de récurrence et comprenant :
- des entrées 61', 62' et 63' aptes à recevoir des signaux EGM acquis sur des dérivations 71', 72' et 73',
- des mémoires tampons 64' permettant d'introduire un temps de retard sur les signaux EGM traités, et
- une fonction de transfert FT apte à traiter les signaux EGM et leurs versions retardées de façon à fournir un signal ECG 90' reconstitué.

En résumé, il apparaît que différentes structures de réseaux temps-retard peuvent être implémentées, en combinaison ou non, avec une approche récurrente.

A titre d'exemple, des approches temps-retard possibles pour un réseau de neurones conforme à l'invention sont :
- FTDNN pour "Focused time-delayed neural networks",
- DTDNN pour "Distributed time-delayed neural networks".

A titre d'exemple également, de telles approches récurrentes sont :
- Elman,
- Hopfield,
- ARX.

On peut aussi citer à titre d'exemple les réseaux temps-retard récurrents de type RTDNN pour "Recurrent time-delayed neural networks", qui combinent à la fois l'approche temps-retard et l'approche récurrente.

A titre d'exemple, des approches temps-retard sont décrites dans K. Hornik and M. Stinchcombe, "Multilayer feedforward networks are universal approximators," Neural Networks, Vol. 2,No. 5 pp. 359-366, 1989 - pour l'approche TDNN - Elman, J.L., "Finding structure in time," Cognitive Science, Vol. 14, pp. 179-211, 1990, pour l'approche ELMAN, ou encore Rodriguez, P., Wiles, J., and Elman, J. L. " A recurrent neural network that learns to count", Connection Science, Vol 11, no 1, pp.5-40, 1999 pour l'approche RTDNN.

Une fois la structure du réseau de neurones temps-retard sélectionnée, deux étapes sont à considérer.

Une première étape, dite d'apprentissage, au cours de laquelle le réseau de neurones détermine une fonction de transfert optimale entre au moins un signal ECG à reconstruire et au moins un signal EGM simultanément enregistrés.

Cet apprentissage peut être effectué lors de la pose de la prothèse ou ultérieurement à la pose de telle sorte que des signaux EGM et ECG soient acquis simultanément.

De fait, l'apprentissage est effectué en enregistrant des EGM et des ECG sur des battements successifs. Le réseau temps-retard effectue alors un apprentissage sur des EGM et des ECG synchrones, ce qui assure une reconstruction ultérieure particulièrement vraisemblable d'un ECG.

De plus, l'apprentissage sur plusieurs battements successifs peut permettre la reconstruction d'ECG à morphologies multiples lorsque ces morphologies multiples sont présentes lors de cet apprentissage.

Au cours de cette première étape, les informations disponibles pour déterminer la fonction de transfert sont au moins un signal EGM et au moins un signal ECG acquis simultanément.

Pratiquement, cette acquisition met en oeuvre les étapes suivantes :
- étape facultative de filtrage passe-bas de l'au moins un signal ECG et de l'au moins un signal EGM à 45 Hz,
- étape facultative de sous échantillonnage des données ECG et EGM, pour réduire les coûts de calcul,
- étape d'optimisation du réseau (nombre de retards, paramètres fixés pour le test de convergence,...).

Dans la réalisation optimale, le dispositif comprend une couche d'entrée avec 15 entrées représentées par trois voies EGM (voie proxA, voie proxVD, voie distVD) à l'instant t et 4 versions retardées avec un pas de 0.01 s (soit un pas d'un échantillon à la fréquence fe de 100Hz, et donc un décalage maximal de 4 échantillons) de chaque voie EGM. La couche cachée du réseau de neurones comprend 50 neurones pour former sa fonction de transfert.

Au cours d'une seconde étape, dite de reconstruction, le réseau de neurones effectue la synthèse du ou des ECG à partir du ou des EGM acquis. Les mêmes dérivations EGM sont utilisées pour cette phase de reconstruction que lors de la phase d'apprentissage.

Dans cette réalisation, le ou les EGM sont enregistrés par la prothèse et le ou les ECG sont reconstruits par application de la fonction de transfert déterminée lors de l'étape d'apprentissage.

Plus précisément, le signal de sortie d'un réseau muni de cette fonction de transfert est un ou plusieurs ECG reconstruits, utilisant la même dérivation que lors de la phase d'apprentissage à partir des étapes suivantes :
- étape facultative de filtrage passe-bas du (ou des) signal EGM à 45 Hz,
- étape facultative de sous-échantillonnage des données EGM lorsque ce sous-échantillonnage a été effectué lors de l'apprentissage,
- application du réseau aux données EGM.

A partir de tels signaux un réseau de neurones du type temps-retard peut reconstruire les douze dérivations d'un ECG.

De façon pratique, un réseau de neurones de ce type a été mis en oeuvre avec 5 neurones pour obtenir ce résultat.

Comme déjà indiqué, un réseau de neurones du type temps-retard peut apprendre une nouvelle fonction de transfert. Il délivre alors de bons résultats en présence de battements nouveaux non appris (extrasystole ventriculaire, échappement jonctionnel, arythmie d'origine ventriculaire,...). En effet, le réseau applique la fonction de transfert qu'il a calculée lors de l'apprentissage et il fournit un résultat vraisemblable. Il a ainsi une capacité de généralisation plus forte.

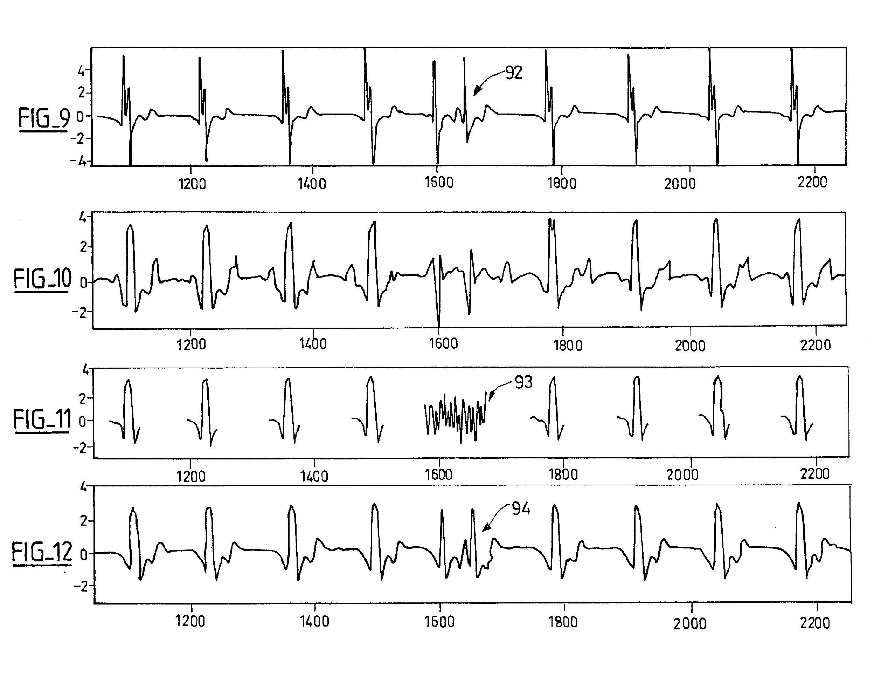

Sur les figures 9, 10, 11 et 12 sont respectivement représentés des signaux EGM (figure 9) ou ECG (figure 10) simultanément acquis, un signal ECG reconstruit (figure 11) selon l'art antérieur et un signal ECG reconstruit selon l'invention (figure 12). En présence de battements non appris 92, le signal ECG 93 reconstruit selon l'art antérieur est inexploitable tandis que le signal ECG reconstruit selon l'invention permet de détecter un battement irrégulier 94.

D'une façon générale, la comparaison des figures 11 et 12 souligne les avantages précédemment mentionnés dans l'utilisation d'un réseau temps-retard ayant effectué l'apprentissage sur des EGM et des ECG synchrones, à savoir :
- une reconstruction particulièrement vraisemblable qui permet de reconstruire un signal continu (P, QRS, T et la ligne de base). Il n'est donc pas nécessaire d'effectuer une étape de détection du QRS. De plus, les problèmes liés au fenêtrage (choix de la largeur de la fenêtre et concaténation des intervalles reconstruits) sont évités, et
- une meilleure résistance aux variations du rythme est obtenue.

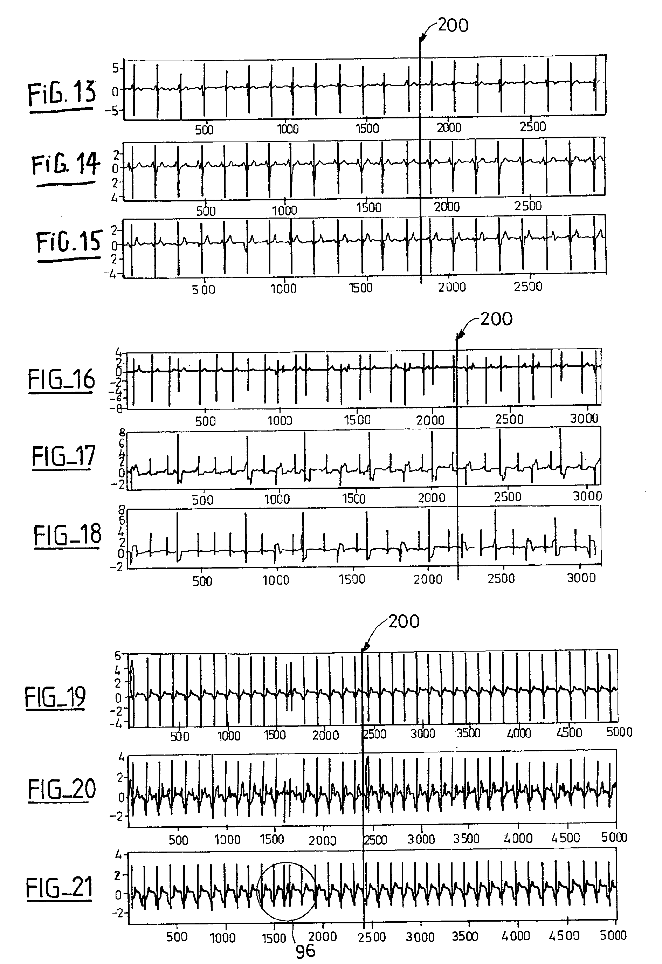

La qualité de la reconstruction est également visible, vis-à-vis de battements d'origine sinusale, en comparant le signal ECG (figure 14) et le signal EGM (figure 13) mesurés au signal ECG reconstruit selon l'invention (figure 15).

Dans le cas d'un patient dont l'ECG contient des battements de morphologies multiples, toutes les morphologies sont reconstruites grâce à un apprentissage sur plusieurs battements successifs comme montré sur les figures 16, 17 et 18.

Finalement, en présence d'une arythmie non apprise (EGM de la figure 19, associé à un ECG mesure à la figure 20), le battement reconstruit selon l'invention (figure 21) est visiblement différent du signal habituel, ce qui permet sa détection.

Il convient de noter que le fait d'effectuer l'apprentissage sur des EGM et ECG synchrones permet de garantir qu'il s'agit bien du même type de battements, une séparation 200 entre la phase d'apprentissage et la phase de détection étant représentée sur ces figures 13 à 21.

De fait, un dispositif conforme à l'invention peut reconstruire un signal continu (P, QRS, T et ligne de base). Il n'est donc pas nécessaire d'effectuer une étape de détection du QRS conformément à l'art antérieur. Ainsi, les problèmes liés au fenêtrage (choix de la largeur de la fenêtre et concaténation des intervalles reconstruits) sont évités.

En outre, l'apprentissage du réseau étant effectué sur plusieurs battements successifs, qui peuvent être de morphologies différentes, il est alors possible de reconstruire des ECG à morphologie multiple.

En d'autres termes, dans le cas d'un patient dont l'ECG contient des morphologies multiples, ces multiples morphologies sont reconstruites grâce à cet apprentissage sur plusieurs battements successifs.

Dans ce cas, le fait que le dispositif effectue un apprentissage sur des EGM et des ECG synchrones permet de fournir une reconstruction particulièrement vraisemblable.

La présente invention est susceptible de nombreuses variantes. Notamment, les signaux EGM peuvent être acquis au moyen d'une pluralité d'électrodes endocavitaires ou, en variante, selon un mode unipolaire tel que le signal EGM est acquis au moyen d'une électrode endocavitaire et d'un boîtier ou de tout autre élément permettant une mesure d'une variation de potentiel au niveau de cette électrode endocavitaire.

Par ailleurs, il est clair que la fréquence d'échantillonnage d'un signal peut varier en fonction des applications.

La présente invention est susceptible de nombreuses variantes. Par exemple, la reconstruction du ou des signaux ECG peut être effectué avec un réseau de neurones à temps de retard, conformément à l'invention, au sein d'un produit implanté ou dans un produit déporté à l'extérieur du corps humain, ce produit déporté recevant les données enregistrées par un produit implanté dans le corps humain.

Dans le cas d'un produit implanté, en ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires.

## Revendications

1. Dispositif médical actif comprenant :
- des moyens d'acquisition d'au moins un signal EGM (EGM₁, EGM₂, EGM₃, EGM"₁, EGM"₂, EGM"₃) d'électrogramme endocavitaire, formé par des échantillons (1, 2, 3, ...200), à partir d'au moins une dérivation (71, 72, 73, 71', 72', 73') endocavitaire ou épicardique, et
- des moyens de calcul d'au moins un signal ECG (ECG₁, ECG₂, ... ECG₁₂) d'électrocardiogramme de surface reconstitué, grâce au traitement des échantillons EGM acquis, par une fonction (FT) de transfert d'un réseau de neurones (60, 60'),
**caractérisé en ce que** le réseau de neurones (60, 60') est un réseau du type temps-retard destiné à traiter simultanément au moins un signal EGM, formé par une première séquence (1, 2, 3, ...100) d'échantillons acquis, et au moins une version retardée de ce signal EGM, formée par une seconde séquence (d+1, d+2, ... d+100) desdits échantillons acquis distincte de la première séquence, de façon à fournir ledit signal ECG reconstitué à partir du signal EGM et de sa version retardée.

2. Dispositif selon la revendication 1 **caractérisé en ce qu'**il comprend des moyens pour déterminer la fonction de transfert (FT) du réseau de neurones (60, 60') en utilisant des acquisitions simultanées, d'une part, de l'au moins un signal EGM et, d'autre part, de l'au moins un signal ECG.

3. Dispositif selon la revendication 2 **caractérisé en ce qu'**il comprend des moyens pour acquérir simultanément l'au moins un signal EGM et l'au moins un signal ECG sur plusieurs battements successifs.

4. Dispositif selon l'une des revendications précédentes **caractérisé en ce qu'**il comprend des moyens pour déterminer la fonction de transfert du réseau de neurones (60, 60') à partir d'au moins deux signaux EGM acquis à l'aide d'au moins deux dérivations (31', 32', 33', 31 ", 32", 33").

5. Dispositif selon la revendication 4 **caractérisé en ce que** le réseau de neurones (60, 60') comprend :
- au moins deux entrées aptes à recevoir les au moins deux signaux EGM acquis sur les au moins deux dérivations (31', 32', 33', 31 ", 32", 33"), et
- une fonction de transfert (FT) apte à traiter lesdits signaux EGM et leurs versions retardées de façon à fournir au moins un signal ECG reconstitué à partir desdits signaux EGM et de leurs dites versions retardées.

6. Dispositif selon la revendication 4 **caractérisé en ce qu'**il comprend des moyens pour déterminer la fonction de transfert (FT) à partir d'au moins deux signaux ECG acquis sur au moins deux dérivations externes.

7. Dispositif selon la revendication 6 **caractérisé en ce que** le réseau de neurones comprend :
- au moins deux entrées aptes à recevoir les signaux EGM acquis sur différentes dérivations (31', 32', 33', 31 ", 32", 33"), et
- une fonction de transfert apte à traiter lesdits signaux EGM et leurs versions retardées de façon à fournir au moins deux signaux ECG reconstitués à partir desdits au moins deux signaux EGM et de leurs versions retardées.

8. Dispositif selon l'une des revendications précédentes **caractérisé en ce qu'**il comprend des moyens pour déterminer la fonction de transfert (FT) en recevant en entrée un groupe de n signaux EGM, issus d'une même dérivation, ce groupe comprenant un signal EGM et n-1 versions différemment retardées dudit signal EGM, où n est compris entre 2 et 50.

9. Dispositif selon l'une des revendications précédentes **caractérisé en ce que** le dispositif comprend des moyens pour acquérir les échantillons d'un signal EGM en effectuant des séquences de mesures avec une fréquence comprise entre 1 Hz et 100 kHz.

10. Dispositif selon la revendication 9 **caractérisé en ce qu'**il comprend des moyens pour enregistrer une séquence sur 1 à 200 battements.

11. Dispositif selon l'une des revendications précédentes **caractérisé en ce qu'**il comprend des moyens pour décaler la première séquence d'échantillons vis-à-vis de la deuxième séquence d'échantillon d'un délai de l'ordre de 0.01s.

12. Dispositif selon l'une des revendications précédentes **caractérisé en ce qu'**il comprend des moyens pour calculer au moins un signal ECG reconstitué de façon continue sur plusieurs battements distincts.

13. Dispositif selon l'une des revendications précédentes **caractérisé en ce que** le réseau de neurones (60, 60') comprend une structure à temps de retard du type *Focused Time-Delayed Neural Network* FTDNN, ou *Distributed Time-Delayed Neural Network* DTDNN.

14. Dispositif selon l'une des revendications précédentes **caractérisé en ce que** le réseau de neurones (60) comprend une structure récurrente.

15. Dispositif selon l'une des revendications précédentes **caractérisé en ce qu'**il comprend des moyens pour que la première séquence (1, 2, 3, ...100) et la seconde séquence (d+1, d+2, ... d+100) comprennent un même nombre d'échantillons temporairement décalés.

## Claims

1. Active medical device comprising:
- means for collecting at least one endocardial electrogram signal EGM (EGM₁, EGM₂, EGM₃, EGM"₁, EGM"₂, EGM"₃), formed by samples (1, 2, 3, ... 200), from at least one endocardial or epicardial derivation (71, 72, 73, 71', 72', 73'), and
- means for calculating at least one electrocardiogram signal ECG (ECG₁, ECG₂, ... ECG₁₂) of reconstructed surface, through the processing of the collected EGM samples, by a transfer function (FT) of a neural network (60, 60'),
**characterized in that** the neural network (60, 60') is a time-delayed network for simultaneously processing at least one EGM signal, formed by a first sequence (1, 2, 3, ... 100) of collected samples, and at least one delayed version of this EGM signal, formed by a second sequence (d+1, d+2, ... d+100) of said collected samples that is distinct from the first sequence, in such a way as to provide said reconstructed ECG signal from the EGM signal and from its delayed version.

2. Device according to Claim 1, **characterized in that** it comprises means for determining the transfer function (FT) of the neural network (60, 60') by using simultaneous acquisitions of, on the one hand, the at least one EGM signal and, on the other hand, the at least one ECG signal.

3. Device according to Claim 2, **characterized in that** it comprises means for simultaneously collecting the at least one EGM signal and the at least one ECG signal over several successive beats.

4. Device according to one of the preceding claims, **characterized in that** it comprises means for determining the transfer function of the neural network (60, 60') from at least two EGM signals collected with the aid of at least two derivations (31', 32', 33', 31", 32", 33").

5. Device according to Claim 4, **characterized in that** the neural network (60, 60') comprises:
- at least two inputs for receiving the at least two EGM signals collected on the at least two derivations (31', 32', 33', 31", 32", 33"), and
- a transfer function (FT) for processing said EGM signals and their delayed versions in such a way as to provide at least one reconstructed ECG signal from said EGM signals and from their said delayed versions.

6. Device according to Claim 4, **characterized in that** it comprises means for determining the transfer function (FT) from at least two ECG signals collected on at least two external derivations.

7. Device according to Claim 6, **characterized in that** the neural network comprises:
- at least two inputs for receiving the EGM signals collected on different derivations (31', 32', 33', 31", 32", 33"), and
- a transfer function for processing said EGM signals and their delayed versions in such a way as to provide at least two reconstructed ECG signals from said at least two EGM signals and from their delayed versions.

8. Device according to one of the preceding claims, **characterized in that** it comprises means for determining the transfer function (FT) by receiving, as input, a group of n EGM signals that come from one and the same derivation, this group comprising an EGM signal and n-1 differently delayed versions of said EGM signal, where n is between 2 and 50.

9. Device according to one of the preceding claims, **characterized in that** the device comprises means for collecting the samples of an EGM signal by performing sequences of measurements with a frequency of between 1 Hz and 100 kHz.

10. Device according to Claim 9, **characterized in that** it comprises means for recording a sequence over 1 to 200 beats.

11. Device according to one of the preceding claims, **characterized in that** it comprises means for shifting the first sequence of samples in relation to the second sequence of samples by a delay of the order of 0.01 s.

12. Device according to one of the preceding claims, **characterized in that** it comprises means for calculating at least one reconstructed ECG signal continuously over several distinct beats.

13. Device according to one of the preceding claims, **characterized in that** the neural network (60, 60') comprises a time-delayed structure such as the *Focused Time-Delayed Neural Network* FTDNN or *Distributed Time-Delayed Neural Network* DTDNN.

14. Device according to one of the preceding claims, **characterized in that** the neural network (60) comprises a recurrent structure.

15. Device according to one of the preceding claims, **characterized in that** it comprises means for ensuring that the first sequence (1, 2, 3, ... 100) and the second sequence (d+1, d+2, ... d+100) comprise the same number of temporarily shifted samples.

## Patentansprüche

1. Aktive medizinische Vorrichtung, mit:
- Mitteln zum Erfassen wenigstens eines Signals EGM (EGM₁, EGM₂, EGM₃, EGM"₁, EGM"₂, EGM"₃) eines endokavitären Elektrogramms, das durch Abtastwerte (1, 2, 3, ..., 200) ausgehend von wenigstens einer endokavitären oder epikardialen Ableitung (71, 72, 73, 71', 72', 73') gebildet ist, und
- Mitteln zum Berechnen wenigstens eines rekonstituierten Signals ECG (ECG₁, ECG₂, ..., ECG₁₂) eines Oberflächen-Elektrokardiogramms durch Bearbeiten der erfassten EGM-Abtastwerte durch eine Übertragungsfunktion (FT) eines neuronalen Netzes (60, 60'),
**dadurch gekennzeichnet, dass** das neuronale Netz (60, 60') ein Netz vom Zeitverzögerungstyp ist, das dazu bestimmt ist, gleichzeitig wenigstens ein EGM-Signal, das durch eine erste Folge (1, 2, 3, ..., 100) erfasster Abtastwerte gebildet ist, und wenigstens eine verzögerte Version dieses EGM-Signals, das durch eine zweite Folge (d + 1, d + 2, ..., d + 100) dieser erfassten Abtastwerte gebildet ist und von der ersten Folge verschieden ist, zu verarbeiten, derart, dass das rekonstituierte ECG-Signal anhand des EGM-Signals und seiner verzögerten Version geliefert wird.

2. Vorrichtung nach Anspruch 1, **dadurch gekennzeichnet, dass** sie Mittel enthält, um die Übertragungsfunktion (FT) des neuronalen Netzes (60, 60') unter Verwendung der gleichzeitigen Erfassungen einerseits des wenigstens einen EGM-Signals und andererseits des wenigstens einen ECG-Signals zu bestimmen.

3. Vorrichtung nach Anspruch 2, **dadurch gekennzeichnet, dass** sie Mittel enthält, um gleichzeitig wenigstens ein EGM-Signal und wenigstens ein ECG-Signal bei mehreren aufeinander folgenden Schlägen zu erfassen.

4. Vorrichtung nach einem der vorhergehenden Ansprüche, **dadurch gekennzeichnet, dass** sie Mittel enthält, um die Übertragungsfunktion des neuronalen Netzes (60, 60') anhand von wenigstens zwei EGM-Signalen, die mit Hilfe wenigstens zweier Ableitungen (31', 32', 33', 31", 32", 33") erfasst werden, zu bestimmen.

5. Vorrichtung nach Anspruch 4, **dadurch gekennzeichnet, dass** das neuronale Netz (60, 60') enthält:
- wenigstens zwei Eingänge, die wenigstens zwei EGM-Signale empfangen können, die an den wenigstens zwei Ableitungen (31', 32', 33', 31", 32", 33") erfasst werden, und
- eine Übertragungsfunktion (FT), die diese EGM-Signale und ihre verzögerten Versionen in der Weise bearbeiten kann, dass wenigstens ein ECG-Signal geliefert wird, das anhand der EGM-Signale und ihrer genannten verzögerten Versionen rekonstituiert wird.

6. Vorrichtung nach Anspruch 4, **dadurch gekennzeichnet, dass** sie Mittel enthält, um die Übertragungsfunktion (FT) anhand wenigstens zweier ECG-Signale, die an wenigstens zwei externen Ableitungen erfasst werden, zu bestimmen.

7. Vorrichtung nach Anspruch 6, **dadurch gekennzeichnet, dass** das neuronale Netz enthält:
- wenigstens zwei Eingänge, die die EGM-Signale, die an verschiedenen Ableitungen (31', 32', 33', 31", 32", 33") erfasst werden, empfangen können, und
- eine Übertragungsfunktion, die diese EGM-Signale und ihre verzögerten Versionen in der Weise verarbeiten kann, dass wenigstens zwei ECG-Signale geliefert werden, die anhand der wenigstens zwei EGM-Signale und ihrer verzögerten Versionen rekonstituiert werden.

8. Vorrichtung nach einem der vorhergehenden Ansprüche, **dadurch gekennzeichnet, dass** sie Mittel enthält, um die Übertragungsfunktion (FT) zu bestimmen, indem am Eingang eine Gruppe von n EGM-Signalen empfangen wird, die von derselben Ableitung stammen, wobei diese Gruppe ein EGM-Signal und n - 1 unterschiedlich verzögerte Versionen des EGM-Signals enthält, wobei n im Bereich von 2 bis 50 liegt.

9. Vorrichtung nach einem der vorhergehenden Ansprüche, **dadurch gekennzeichnet, dass** die Vorrichtung Mittel enthält, um die Abtastwerte eines EGM-Signals zu erfassen, indem Folgen von Messungen mit einer Frequenz im Bereich von 1 Hz bis 100 kHz ausgeführt werden.

10. Vorrichtung nach Anspruch 9, **dadurch gekennzeichnet, dass** sie Mittel enthält, um eine Folge bei 1 bis 200 Schlägen aufzuzeichnen.

11. Vorrichtung nach einem der vorhergehenden Ansprüche, **dadurch gekennzeichnet, dass** sie Mittel enthält, um die erste Folge von Abtastwerten in Bezug auf die zweite Folge von Abtastwerten um eine Verzögerung in der Größenordnung von 0,01 s zu verschieben.

12. Vorrichtung nach einem der vorhergehenden Ansprüche, **dadurch gekennzeichnet, dass** sie Mittel enthält, um wenigstens ein ECG-Signal zu berechnen, das auf kontinuierliche Weise an mehreren verschiedenen Schlägen rekonstituiert wird.

13. Vorrichtung nach einem der vorhergehenden Ansprüche, **dadurch gekennzeichnet, dass** das neuronale Netz (60, 60') eine Zeitverzögerungsstruktur des Typs *Focused Time-Delayed Neural Network FTDNN* oder *Distributed Time-Delayed Neural Network DTDNN* enthält.

14. Vorrichtung nach einem der vorhergehenden Ansprüche, **dadurch gekennzeichnet, dass** das neuronale Netz (60) eine rekurrente Struktur aufweist.

15. Vorrichtung nach einem der vorhergehenden Ansprüche, **dadurch gekennzeichnet, dass** sie Mittel enthält, damit die erste Folge (1, 2, 3, ..., 100) und die zweite Folge (d + 1, d + 2, ..., d + 100) dieselbe Anzahl von zeitlich verschobenen Abtastwerten enthalten.
